# EUROPEAN PATENT APPLICATION

(11) **EP 4 378 403 A1**
(43) Date of publication of application: **05.06.2024**
(21) Application number: 21959085.8
(22) Date of filing: 02.11.2021
(51) Int. Cl.: A61B 17/22, A61B 17/225

(54) **COMBINED CONTROL THROMBECTOMY DEVICE**

(30) Priority: 29.09.2021 CN 202111151414
(71) Applicant: Beijing Taijieweiye Technology Co., Ltd., Beijing 101204 (CN)
(72) Inventor: CHENG, Wenjie, Beijing 101204 (CN); MU, Lei, Beijing 101204 (CN); XU, Yongsong, Beijing 101204 (CN); WANG, Mengjing, Beijing 101204 (CN); GUO, Leichen, Beijing 101204 (CN); FAN, Wenji, Beijing 101204 (CN); WU, Yongzhao, Beijing 101204 (CN); MA, Lili, Beijing 101204 (CN)
(74) Representative: Glück Kritzenberger Patentanwälte PartGmbB
(86) International application number: PCT/CN2021/128045
(87) International publication number: WO 2023/050521

(57) **Abstract**

A combined adjustable thrombectomy device, comprising: a control unit (1); a delivery unit (2), comprising an outer tube (21), an inner tube (22), a control core wire (23), and a guide spring (24), the inner tube (22) being inserted in the outer tube (21), having a proximal end connected to the control unit (1), and being configured to slide in the outer tube (21) under the adjustment of the control unit (1), and the control core wire (23) penetrating through the inner tube (22), having a proximal end connected to the control unit (1), and being configured to slide in the inner tube (22) under the adjustment of the control unit (1); a collection net basket (3), having a proximal end fixedly connected to a distal end of the outer tube (21); and an adjustable net ball (4), having a proximal end fixedly connected to a distal end of the inner tube (22), and a distal end connected to a proximal end of the guide spring (24). The control core wire (23) penetrates the adjustable net ball (4), and has a distal end fixedly connected to the proximal end of the guide spring (24). When in use, the inner tube (22) is adjusted by rotating or pushing an adjusting button (12) so as to adjust the position of the adjustable net ball (4); in addition, the control core wire (23) is adjusted so that the adjustable net ball (4) can expand and open after passing through a lesion part, thereby achieving the capture of thrombus (6) and removal thereof along with the device.

## Description

The application claims the priority to Chinese Patent Application No. 202111151414.0 filed with the Patent Office of the People's Republic of China on September 29, 2021 and entitled "Combined Adjustable Thrombectomy Device".

### BACKGROUND OF THE INVENTION

### 1. Technical Field

The present disclosure relates to the field of medical instruments, in particular to a combined adjustable thrombectomy device.

### 2. Description of Related Art

Cerebral apoplexy is a kind of acute cerebrovascular diseases, including ischemic stroke (blood cannot flow into the brain as a result of vascular obstruction) and hemorrhagic stroke (sudden rupture of cerebral vessels). The cerebral apoplexy has features of high incidence rate, high disability rate, high recurrence rate, high fatality rate, and high cost. China has become the first country all over the world where apoplexy happens. Apoplexy disease burden in China is in a situation of explosive growth and has the trend of young age, relapse, multiple, common disease and the like.

The first batch of therapeutic method for acute ischemic stroke (AIS) is intravenous injection (IV) of a tissue plasminogen activator (tPA) for thrombolysis. The therapeutic outcome shows that although the incidence rate of symptomatic intracranial hemorrhage (sICH) increases slightly, intravenous injection of tPA within three hours after symptom emergence may improve the functional independence in the third month. However, the success rate to achieve revascularization by way of thrombolysis is quite low, which accelerates the development of mechanical thrombectomy and promotes the emergence of a large batch of thrombectomy devices.

However, most thrombectomy devices on the market are embedded into thrombi through stents, and then the stents and the thrombi are taken out together. For example, a Solitaire thrombectomy stent from Medtronic, segmented thrombectomy stents Embotrap serial stents from Johnson, an adjustable Tigertriever stent from Rapid Medical and the like. On the one hand, in the process of embedding these stents into the thrombi, the thrombi are easily ruptured, so that thrombus fragments escape and flow to the distal ends of blood vessels along with blood to form new thrombi. On the other hand, the Solitaire and Embotrap stents are laser engraving self-expanding stents, and in the thrombectomy process, they are likely to damage the vascular walls. In addition, it takes a certain time to embed the laser engraving self-expanding stents into the thrombi and the thrombi cannot be taken out of the body immediately, so that the risk that a patient is disable and even dead is improved. Although the Tigertriever stent which is a woven adjustable stent is easy to adjust and operate and short of a precise dimension adjusting function, the size of the stent is easily over-adjusted. When the size of the stent is much larger than an expected blood vessel, the blood vessel will be severely damaged, thereby bringing a hemorrhage risk.

### BRIEF SUMMARY OF THE INVENTION

To overcome defects in the prior art, the object of the present disclosure is to provide a combined adjustable thrombectomy device to at least partially solve the above technical problems.

In order to achieve the above object, the present disclosure provides a combined adjustable thrombectomy device, including:
a control unit;
a delivery unit comprising an outer tube, an inner tube, a control core wire and a guide spring, wherein the inner tube is inserted in the outer tube, the inner tube has a proximal end connected to the control unit, and the inner tube is configured to slide in the outer tube under the adjustment of the control unit; and the control core wire penetrates through the inner tube, the control core wire has a proximal end connected to the control unit, and the control core wire is configured to slide in the inner tube under the adjustment of the control unit;
a collection net basket having a proximal end fixedly connected to a distal end of the outer tube; and
an adjustable net ball having a proximal end fixedly connected to a distal end of the inner tube and a distal end connected to a proximal end of the guide spring, wherein the control core wire penetrates through the adjustable net ball and has a distal end fixedly connected to the proximal end of the guide spring, so that the adjustable net ball can expand or contract under the action of the control core wire.

In some solutions, the control unit includes an outer shell and a sliding mechanism; and
an adjusting button is arranged on the outer shell.

In some solutions, a scale designation is arranged on the outer shell and is configured to display position information of the adjusting button.

In some solutions, the sliding mechanism is a direct connecting mechanism, a spiral mechanism, a gear-rack mechanism, a ratchet mechanism or a connecting rod mechanism.

In some solutions, the adjusting mode of the adjusting button includes sliding, rotating or pulling.

In some solutions, the adjusting button includes a first adjusting button and a second adjusting button;
the first adjusting button is connected to the proximal end of the inner tube through the sliding mechanism and is configured to adjust relative positions of the adjustable net ball and the collection net basket; and
the second adjusting button is connected to the proximal end of the control core wire through the sliding mechanism and is configured to adjust expansion or contraction of the adjustable net ball.

In some solutions, the collection net basket is a single-layered net ball or a multi-layered net ball; and
a pore diameter of a mesh at the proximal end of the collection net basket is greater than a pore diameter of a mesh at the distal end of the collection net basket.

In some solutions, the collection net basket is interwoven by nickel-titanium metal wires or cobalt-chromium alloy wires; and
the collection net basket is cylindrical in a compressed state.

In some solutions, the adjustable net ball is interwoven by nickel-titanium metal wires or cobalt-chromium alloy wires;
the adjustable net ball is cylindrical in a compressed state; and
the adjustable net ball is spherical, ellipsoidal, disc-like, cone-shaped, cylindrical or sausage-shaped in an expanded state.

In some solutions, the section of the nickel-titanium metal wires is round, elliptical or square.

In some solutions, the guide spring is wound by the nickel-titanium alloy wires; and
a ball cap structure is arranged at the distal end of the guide spring.

In some solutions, a first developing mark is arranged at the proximal end of the collection net basket;
a second developing mark is arranged at the proximal end of the adjustable net ball; and
a third developing mark is arranged at the distal end of the adjustable net ball.

In some solutions, the adjustable net ball is a single net ball or a plurality of net balls connected in series.

The embodiment of the present disclosure provides a combined adjustable thrombectomy device. The control core wire of the delivery unit is adjusted through the adjusting button of the control unit, so that the adjustable ball net penetrating through the thrombus is expanded and opened. Through continuous adjustment by the adjusting button, the adjustable net ball and the captured thrombus are retreated to the collection net basket and are retreated together out of the body. Through the segmented thrombectomy device, it is unnecessary to match the length of the thrombus, and meanwhile, the stimulation to the blood vessel can be reduced, so that a secondary thrombus during retreat can be prevented.

### BRIEF DESCRIPTION OF THE SEVERAL VIEWS OF THE DRAWINGS

FIG. 1 is a schematic structural diagram of a combined adjustable thrombectomy device according to an embodiment of the present disclosure;
FIG. 2 is a schematic diagram of a collection net basket and an adjustable net ball in a compressed state according to an embodiment of the present disclosure;
FIG. 3 is a schematic diagram of the collection net basket and the adjustable net ball in an unfolded state according to an embodiment of the present disclosure;
FIG. 4 is a schematic diagram of relative positions of the collection net basket and the adjustable net ball according to an embodiment of the present disclosure;
FIG. 5 is a schematic diagram of the adjustable net ball completely recovered according to an embodiment of the present disclosure;
FIG. 6 is a schematic diagram of a sectional structure of a double-layered collection net basket according to an embodiment of the present disclosure;
FIG. 7 is a schematic diagram of a sectional structure of a single-layered collection net basket according to an embodiment of the present disclosure;
FIG. 8 is a schematic diagram showing a mesh density of a woven structure of the collection net basket according to an embodiment of the present disclosure;
FIG. 9 is a schematic diagram of a plane structure path of a weaving path of metal wires of the collection net basket and the adjustable net ball according to an embodiment of the present disclosure;
FIG. 10 is a first schematic structural diagram of the adjustable net ball according to an embodiment of the present disclosure;
FIG. 11 is a second schematic structural diagram of the adjustable net ball according to an embodiment of the present disclosure;
FIG. 12 is a third schematic structural diagram of the adjustable net ball according to an embodiment of the present disclosure;
FIG. 13 is a fourth schematic structural diagram of the adjustable net ball according to an embodiment of the present disclosure;
FIG. 14 is a fifth schematic structural diagram of the adjustable net ball according to an embodiment of the present disclosure;
FIG. 15a is a sixth schematic structural diagram of the adjustable net ball according to an embodiment of the present disclosure;
FIG. 15b is a schematic diagram of an open state of the sixth schematic structural diagram of the adjustable net ball according to an embodiment of the present disclosure;
FIG. 16a is a first working schematic diagram of a combined adjustable thrombectomy device according to an embodiment of the present disclosure;
FIG. 16b is a second working schematic diagram of a combined adjustable thrombectomy device according to an embodiment of the present disclosure;
FIG. 16c is a third working schematic diagram of a combined adjustable thrombectomy device according to an embodiment of the present disclosure;
FIG. 16d is a fourth working schematic diagram of a combined adjustable thrombectomy device according to an embodiment of the present disclosure; and
FIG. 16e is a fifth working schematic diagram of a combined adjustable thrombectomy device according to an embodiment of the present disclosure.

### DETAILED DESCRIPTION OF THE INVENTION

The technical solution of the present disclosure will be further described in detail below in combination with drawings and embodiments.

The embodiment of the present disclosure provides a combined adjustable thrombectomy device, including: a control unit; a delivery unit including an outer tube, an inner tube, a control core wire, and a guide spring, the inner tube being capable of sliding in the outer tube, the control core wire sliding in the inner tube, a proximal end of the collection net basket being connected to a distal end of the outer tube, and a proximal end of the adjustable net ball being fixedly connected to a distal end of the inner tube; and the control unit includes an adjusting button, the inner tube being adjusted by the adjusting button to achieve position adjustment of the adjustable net ball, and the control core wire being adjusted by the adjusting button to achieve state adjustment of the adjustable net ball; the adjustable net ball expands to capture the thrombus, retreats the thrombus back to the collection net basket and retreats the thrombus together with the collection net basket back out of the body. Through the segmented thrombectomy device, it is unnecessary to match the length of the thrombus, and meanwhile, the stimulation to the blood vessel can be reduced, so that a secondary thrombus during retreat can be prevented.

As discussed herein, the term "distal end" or "proximal end" are used for describing the position or direction relative to a handheld end of a treating physician or a medical interventional physician hereafter. The "distal end" is the position away from the direction of the handheld end of the physician or the interventional physician. The "proximal end" is the position close to the direction of the handheld end of the physician or the interventional physician. The present disclosure is described in detail below through specific embodiments. It shall be understood that the embodiments below are not intended to limit the present disclosure. Those skilled in the art are capable of thinking of arranging and combining specific features in the embodiments to form other similar solutions based on the concept of the present disclosure.

As discussed herein, the term "connect" may refer to direct connection of two elements or indirect connection through an intermediate. The two modes shall be construed as the content included by "connect".

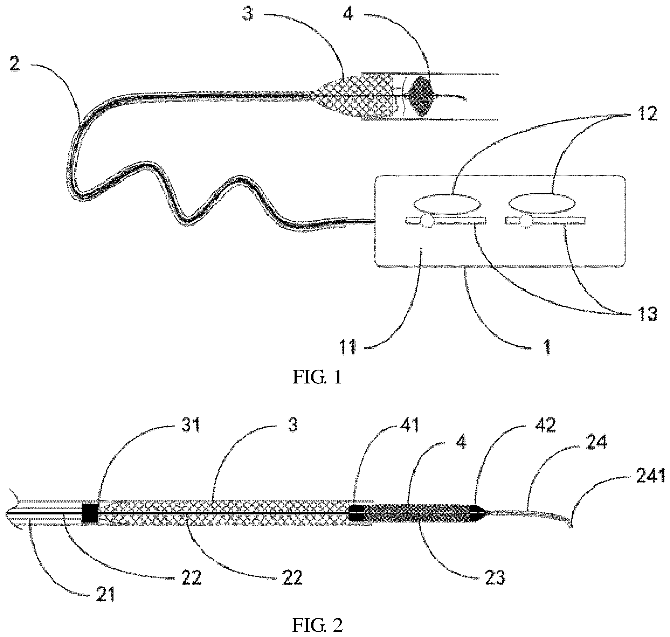

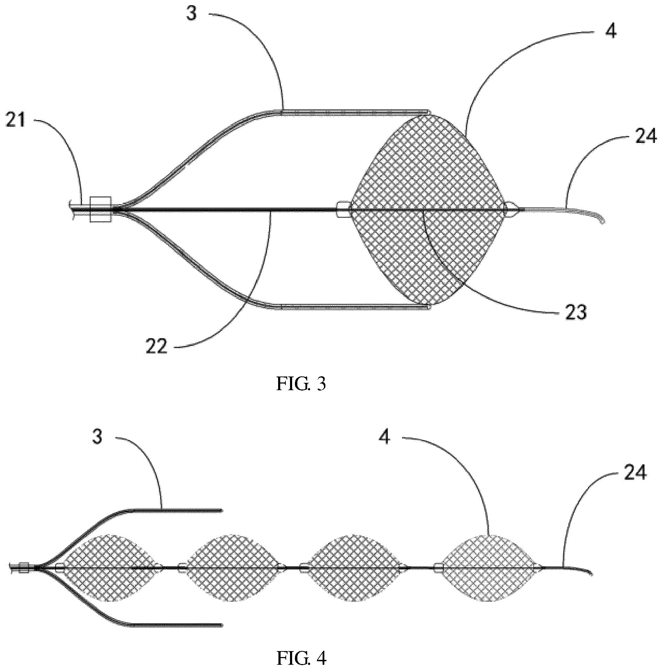

FIG. 1 is a schematic structural diagram of a combined adjustable thrombectomy device according to an embodiment of the present disclosure; FIG. 2 is a schematic diagram of a collection net basket and an adjustable net ball in a compressed state according to an embodiment of the present disclosure; and FIG. 3 is a schematic diagram of the collection net basket and the adjustable net ball in an unfolded state according to an embodiment of the present disclosure. As shown in FIGs. 1-3, the combined adjustable thrombectomy device includes a control unit 1, a delivery unit 2, a collection net basket 3 and an adjustable net ball 4.

The control unit 1 includes an outer shell 11 and a sliding mechanism (not drawn in the drawings); and an adjusting button 12 is arranged on the outer shell 11. The adjusting button 12 includes a first adjusting button and a second adjusting button, and meanwhile, a scale designation 13 is further arranged on the outer shell 11 and is configured to display position information of the adjusting button 12.

The delivery unit 2 includes an outer tube 21, an inner tube 22, a control core wire 23, and a guide spring 24, wherein the inner tube 22 is inserted in the outer tube 21, the inner tube 22 has a proximal end connected to the adjusting button 12 through a sliding mechanism, and the inner tube 22 is capable of sliding in the outer tube 21; the adjusting button 12 adjusts the inner tube 22 through the sliding mechanism, so that the inner tube 22 is capable of sliding in the outer tube 21; the control core wire 23 penetrates through the inner tube 22, the control core wire 23 has a proximal end connected to the adjusting button 12 through the sliding mechanism, and the control core wire 23 is capable of sliding in the inner tube 22; the adjusting button 12 adjusts the control core wire 23 through the sliding mechanism, so that the control core wire 23 is capable of sliding in the inner tube 22.

The distal end of the collection net basket 3 has a first opening, a first developing mark 31 is arranged at the proximal end of the collection net basket 3, and the proximal end of the collection net basket 3 is fixedly connected to the distal end of the outer tube 21, so that the collection net basket 3 is capable of moving to a corresponding position in a blood vessel under the action of the outer tube 21.

A second developing mark 41 is arranged at the proximal end of the adjustable net ball 4, the proximal end of the adjustable net ball 4 is fixedly connected to the distal end of the inner tube 22 with a fixed connection mode of riveting or adhering, a third developing mark 42 is arranged at the distal end of the adjustable net ball 4, and the distal end of the adjustable net ball 4 is connected to the proximal end of the guide spring 24.The control core wire 23 is inserted in the adjustable net ball 4, and the distal end of the control core wire 23 and the distal end of the third developing mark 42 are fixedly connected to the proximal end of the guide spring 24, so that the adjustable net ball 4 expands or contracts when the control core wire 23 slides.

The sliding mechanism which is a motion conversion mechanism can be a direct connection structure, a spiral structure, a gear-rack structure, a ratchet mechanism or a connecting rod mechanism. The sliding mechanism is capable of converting adjustment of the adjusting button 12 into movement of the inner tube 22 or the control core wire 23, so as to adjust the position and state of the adjustable net ball 4. The adjusting mode of the adjusting button 12 can be sliding, rotating or pulling.

Specifically, the adjusting button 12 includes the first adjusting button and the second adjusting button. The first adjusting button is connected to the proximal end of the inner tube 22 through the sliding mechanism, and the sliding mechanism adjusts the inner tube 22 to adjust the position of the collection net basket 3 in the blood vessel; the second adjusting button is connected to the proximal end of the control core wire 23 through the sliding mechanism, and the sliding mechanism adjusts the control core wire 23 to operate the state of the adjustable net ball 4, so that the adjustable net ball 4 expands or contracts.

FIG. 4 is a schematic diagram of relative positions of the collection net basket and the adjustable net ball according to an embodiment of the present disclosure. As shown in FIG. 4, the scope of the adjustable net ball 4 capable of movably stretching relative to the collection net basket 3 is shown in the figure. By adjusting the position of the adjustable net ball 4 relative to the collection net basket 3, the combined adjustable thrombectomy device is capable of being adaptive to thrombi with different lengths, so as to grab the thrombi with different lengths.

The collection net basket 3 can be a single-layered net ball or a multi-layered net ball. The collection net basket 3 is in a compressed state during delivery, and is capable of expanding automatically to open when being pushed out from the conduit. The collection net ball 3 is formed by interweaving the metal wires 33 and is cylindrical in the compressed state. The weaving structure at the distal end of the collection net basket 3 is denser and that at the proximal end thereof is sparser. The pore diameter of a mesh at the proximal end of the collection net basket 3 is greater than that of a mesh at the distal end of the collection net basket 3. When the collection net basket 3 is the single-layered net ball, a return structure 32 is arranged at the first opening to reduce stimulation of the metal wires to the blood vessel. When the collection net basket 3 is multi-layered net basket, it can be of a U-shaped encircling structure, so that the inner wall of the blood vessel is prevented from being stimulated at the opening of the net basket.

The adjustable net ball 4 is formed by interweaving the metal wires 33, and is cylindrical in the compressed state. The adjustable net ball 4 expands to be spherical, ellipsoidal, disc-like, cone-shaped, cylindrical or sausage-shaped under adjustment of the adjusting button 12. The adjustable net ball 4 can be a single net ball or can be formed by connecting a plurality of net balls in series.

The guide spring 24 is a flexible segment of a spring structure formed by winding a nickel-titanium alloy wire, and a flexible ball cap structure 241 is arranged at the distal end of the guide spring 24.

The first developing mark 31, the second developing mark 41 and the third developing mark 42 according to an embodiment of the present disclosure are X-ray radiopaque marks. During work, the positions of the collection net basket 3 and the adjustable net ball 4 can be observed through the developing marks. Meanwhile, the metal wires used for weaving the collection net basket 3 and the adjustable net ball 4 are developing wires, so that the working states of the collection net basket 3 and the adjustable net ball 4 can be observed under X-ray.

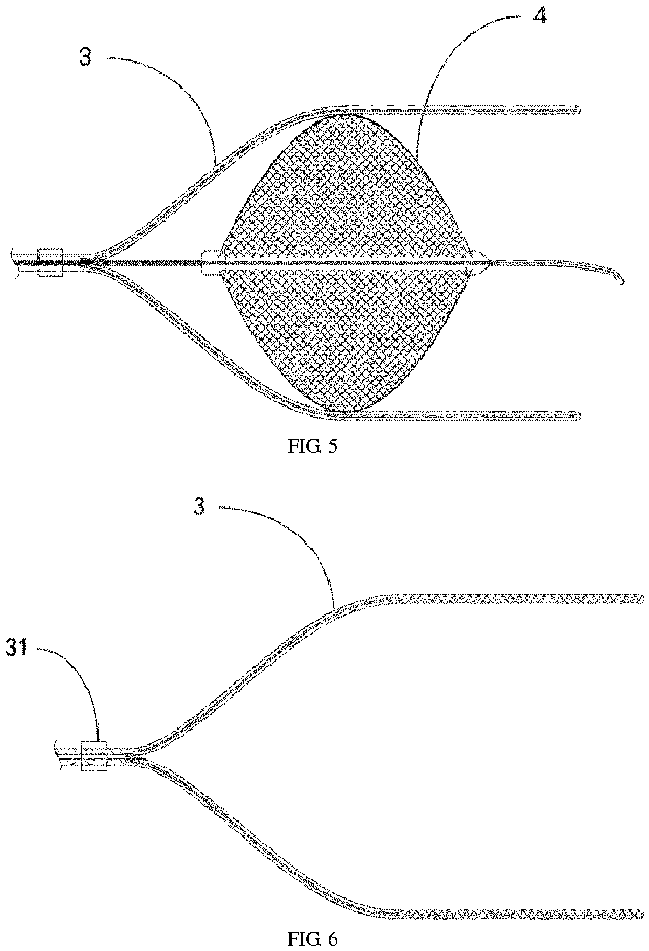

FIG. 5 is a schematic diagram of the adjustable net ball completely recovered according to an embodiment of the present disclosure. As shown in FIG. 5, the adjustable net ball 4 can be observed through the second developing mark 41 and the third developing mark 42 when being recovered into the collection net basket 3, so that the specific position of the adjustable net ball 4 in the collection net basket 3 can be observed, and the device can be smoothly recovered after the device captures the thrombus.

FIG. 6 is a schematic diagram of a sectional structure of a double-layered collection net basket according to an embodiment of the present disclosure. As shown in FIG. 6, the distal end of the double-layered collection net basket 3 is designed in arc shape, so that stimulation of the distal end of the collection net basket 3 to the inner wall of the blood vessel can be reduced, and the blood vessel can be better protected.

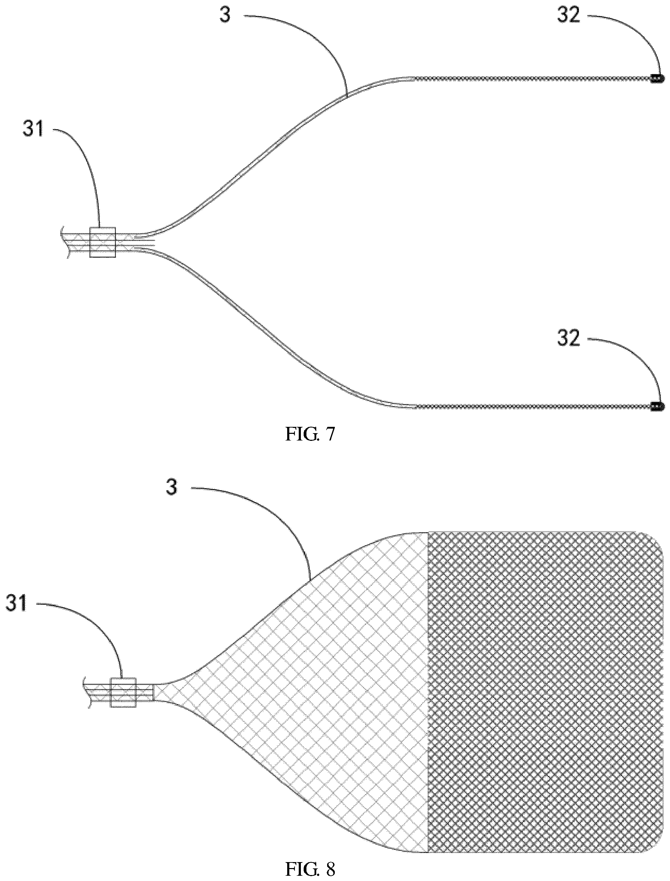

FIG. 7 is a schematic diagram of a sectional structure of a single-layered collection net basket according to an embodiment of the present disclosure. As shown in FIG. 7, the distal end of the single-layered collection net basket 3 is of a dense mesh U-shaped structure, so that stimulation of the distal end of the collection net basket 3 to the inner wall of the blood vessel can be reduced, and the blood vessel can be better protected; and meanwhile, the single-layered collection net basket 3 is smaller in size, so that the structural size of the integral delivery unit 2 can be reduced, thereby facilitating use thereof in a narrower blood vessel. The U-shaped structure at the distal end has the developing action, thereby facilitating observation of the distal end of the collection net basket 3.

FIG. 8 is a schematic diagram showing a mesh density of a woven structure of the collection net basket according to an embodiment of the present disclosure. As shown in FIG. 8, meshes at the proximal end of the collection net basket 3 are sparser and meshes at the distal end thereof are denser; the sparser meshes at the proximal end facilitate passing of thrombus, so that a negative pressure suction device sucks the thrombus out, and cutting of the thrombus can be reduced by the sparser meshes, so that the risk of thrombus rupture is reduced; and the denser meshes at the distal end are capable of preventing thrombus escape.

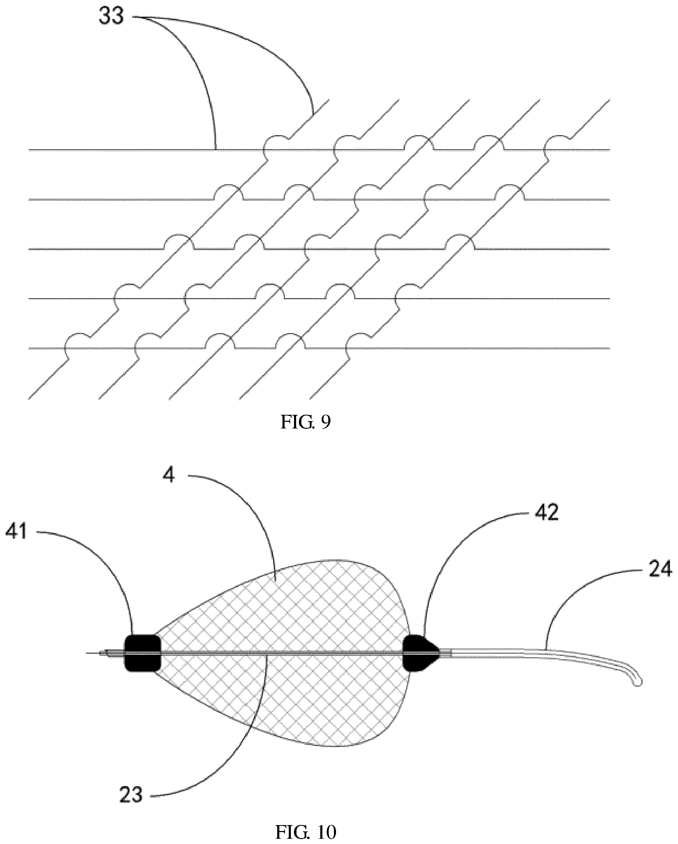

FIG. 9 is a schematic diagram of a plane structure path of a weaving path of metal wires of the collection net basket and the adjustable net ball according to an embodiment of the present disclosure. The collection net basket 3 and the adjustable net ball 4 both are formed by weaving the metal wires 33, where the metal wires 33 can be nickel-titanium metal wires or cobalt-chromium alloy wires. The section of the nickel-titanium metal wires can be round, elliptical or square. The weaving mode of the metal wires 33 is an interweaving mode. As shown in FIG. 9, a two-pressing-two interweaving mode is used in FIG. 9, i.e., a mode of interweaving every two metal wires 33. A one-pressing-one or three-pressing-three interweaving mode can be also used. The mesh structures and radial forces of the collection net baskets 3 obtained by interweaving different quantities of metal wires are different, and technicians can adjust the interweaving mode according to actual demands.

FIG. 10 is a first schematic structural diagram of the adjustable net ball according to an embodiment of the present disclosure. As shown in FIG. 10, the adjustable net ball 4 is of a yali pear structure which is embedded into the thrombus more favorably, and has a larger contact area with the thrombus, thereby further facilitating separation of the thrombus from the vascular wall. Meanwhile, the proximal end of the structure is smaller, so that the structure enters the collection net basket 3 more favorably during thrombus recovery.

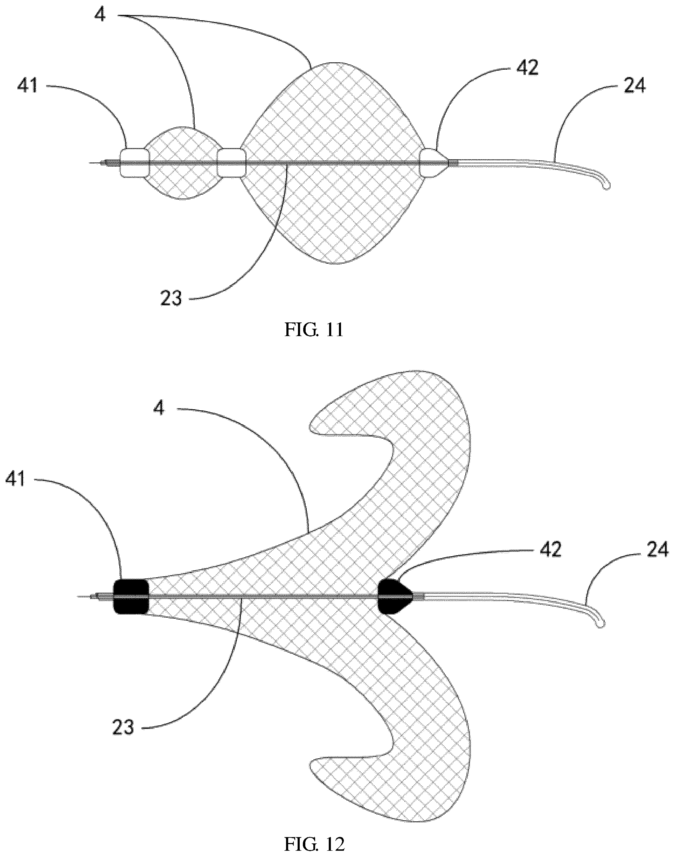

FIG. 11 is a second schematic structural diagram of the adjustable net ball according to an embodiment of the present disclosure. As shown in FIG. 11, the adjustable net ball 4 of the structure includes a small ball structure at the proximal end and a large ball structure at the distal end. The small ball structure is embedded into the thrombus more favorably and plays a role of fixing the thrombus, and the large ball structure at the distal end is capable of better preventing the thrombus from escaping.

FIG. 12 is a third schematic structural diagram of the adjustable net ball according to an embodiment of the present disclosure. As shown in FIG. 12, the section of the adjustable net ball 4 is a section with fishhooks on both sides. The adjustable net ball 4 of the structure is capable of forming an embedding structure with the thrombus during thrombus collection. The fishhook structure is capable of better peeling off the thrombus on the inner wall of the blood vessel, and is also capable of recovering thrombus fragments so as to prevent fragmented thrombi from escaping.

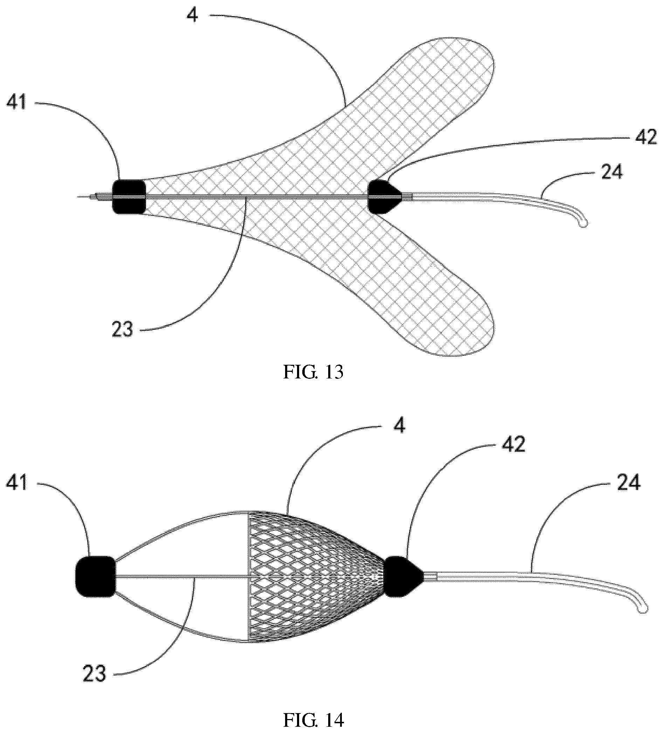

FIG. 13 is a fourth schematic structural diagram of the adjustable net ball according to an embodiment of the present disclosure. As shown in FIG. 13, the adjustable net ball 4 of the structure is capable of better fitting the blood vessel, reducing the internal frictional force between the adjustable net ball 4 and the blood vessel, and effectively separating the thrombus from the blood vessel and pulling the thrombus into the collection net basket 3.

FIG. 14 is a fifth schematic structural diagram of the adjustable net ball according to an embodiment of the present disclosure. As shown in FIG. 14, the adjustable net ball 4 of the structure is of a handbasket structure with an opening at the proximal end and a dense net structure at the distal end, is capable of being attached to the inner wall of the blood vessel after expansion, and forms a whole of the thrombus and the adjustable net ball 4, so that a lasso type sliding device is formed between the thrombus and the adjustable net ball 4, which is more favorable to peel off the thrombus from the blood vessel, and therefore, stimulation of the adjustable net ball 4 to the inner wall of the blood vessel can be effectively reduced.

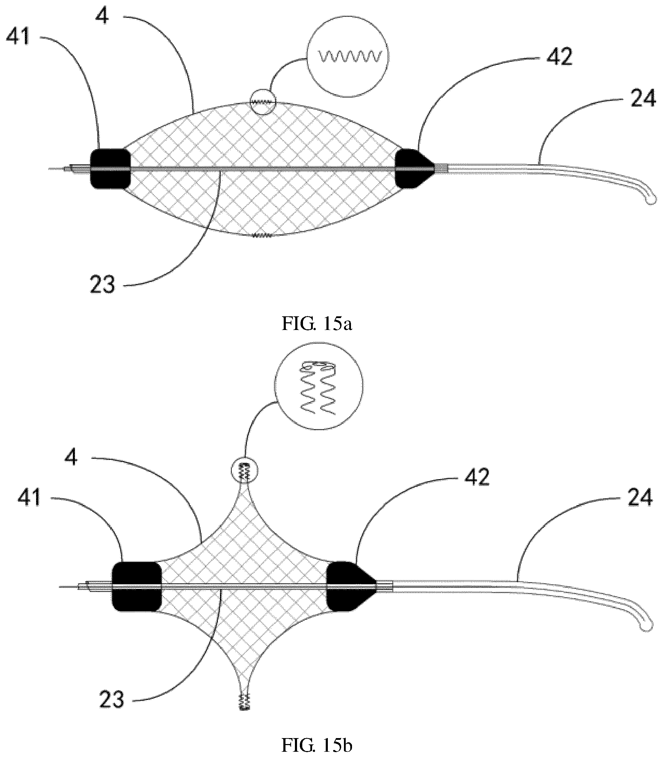

FIG. 15a is a sixth schematic structural diagram of the adjustable net ball according to an embodiment of the present disclosure; and FIG. 15b is a schematic diagram of an open state of the sixth schematic structural diagram of the adjustable net ball according to an embodiment of the present disclosure. As shown in FIG. 15a and FIG. 15b, combined with a spring type hinge mechanism in the structure, a sunken net disc structure is formed after the adjustable net ball 4 is opened, so that the adjustable net ball 4 and the hinge mechanism form a corresponding thrombus collection adjustable net ball. A plurality of hinge mechanisms has the advantage of controlling change of structural morphology of the adjustable net ball 4 in a limited manner through connecting points of the hinges.

In the combined adjustable thrombectomy device according to an embodiment of the present disclosure, the outer diameter of the outer tube 21 is 0.3-2 mm, and the inner diameter thereof is 0.2-1.8 mm; the outer diameter of the inner tube 22 is 0.2-1.6 mm, and the inner diameter thereof is 0.1-1.4 mm; the diameter of the control core wire 23 is 0.08-1 mm; the outer diameter of the collection net basket 3 is 1-10 mm; the diameter of the weaving metal wires for the collection net basket 3 is 0.01-0.16 mm; the outer diameter of the adjustable net ball 4 is 0.6-10 mm; and the diameter of the weaving metal wires for the adjustable net ball 4 is 0.01-0.16 mm.

The specific working process of the combined adjustable thrombectomy device provided by the embodiments of the present disclosure is carried out in the following way.

The adjusting button 12 is rotated or pushed and pulled to pull the control core wire 23, and the control core wire 23 tensions the adjustable net ball 4, so that the adjustable net ball 4 expands and opens; then the adjusting button 12 controls the inner tube to retreat the adjustable net ball 4, the captured thrombus is collected to the collection net basket 3 and they are together retreated to the conduit, and then they are integrally retreated out of the body, so that the thrombus in the blood vessel is taken out.

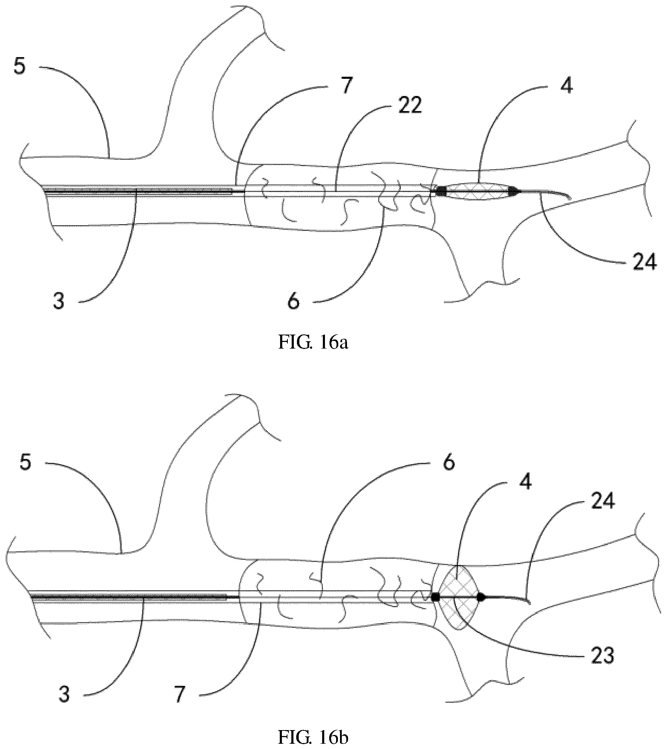

Specifically, FIG. 16a-FIG. 16e are working schematic diagrams of a combined adjustable thrombectomy device according to an embodiment of the present disclosure. As shown in the FIG. 16a-FIG. 16e, in FIG. 16a, the conduit 7 is a delivery conduit used in common interventional therapy. The thrombectomy device is delivered to the thrombus 6 in the blood vessel 5 through the conduit 7. By observing the developing mark, after the distal end of the conduit 7 penetrates through the thrombus 6, the adjusting button 12 adjusts the inner tube 22 to push the adjustable net ball 4 out of the conduit 7.

In FIG. 16b, the adjusting button 12 adjusts the control core wire 23, so that the adjustable net ball 4 expands in the original site. Meanwhile, the conduit 7 is retreated.

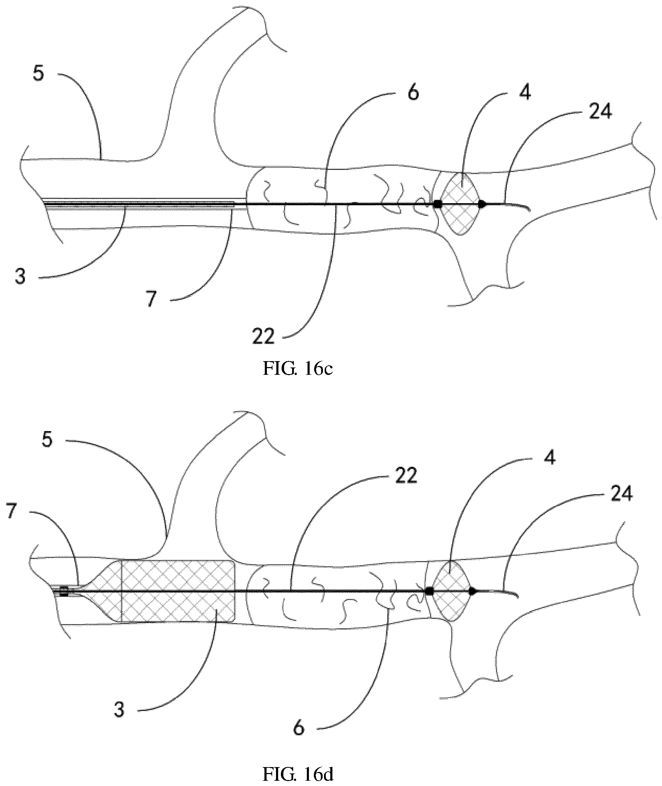

In FIG. 16c, the conduit 7 is retreated to the proximal end of the thrombus 6. The collection net basket 3 is still located in the conduit 7, and is in an unopened state.

In FIG. 16d, the conduit 7 is continuously retreated to the proximal end of collection net basket 3, so that the collection net basket 3 is completely exposed in the blood vessel 5. At this time, the collection net basket 3 is opened automatically to capture the thrombus 6 together with the adjustable net ball 4.

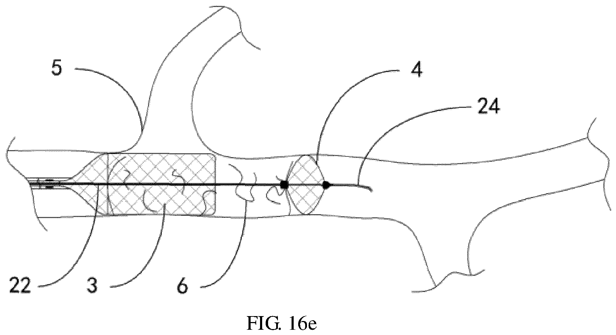

In FIG. 16e, the adjusting button 12 adjusts the inner tube 22, so that it drives the adjustable net ball 4 to retreat, the captured thrombus 6 is collected in the collection net basket 3, and the thrombus is retreated together with the collection net basket 3 out of the body.

The embodiment of the present disclosure provides a combined adjustable thrombectomy device. The control core wire of the delivery unit is adjusted through the adjusting button of the control unit, so that the adjustable ball net penetrating through the thrombus is expanded and opened. Through continuous adjustment by the adjusting button, the adjustable net ball and the captured thrombus are retreated to the collection net basket and are retreated together out of the body. Through the segmented thrombectomy device, it is unnecessary to match the length of the thrombus, and meanwhile, the stimulation to the blood vessel is reduced, so that a secondary thrombus during retreat is prevented.

The above specific implementations further describe the objects, technical solutions and beneficial effects of the present disclosure. It shall be understood that the above is merely the specific implementations of the present disclosure and is not used to limit the scope of protection of the present disclosure. Any modification, equivalent substitution, improvements and etc. made within the spirit and principle of the present disclosure shall fall within the scope of protection of the present disclosure.

## Claims

1. A combined adjustable thrombectomy device, comprising:
a control unit;
a delivery unit comprising an outer tube, an inner tube, a control core wire and a guide spring, wherein the inner tube is inserted in the outer tube, the inner tube has a proximal end connected to the control unit, and the inner tube is configured to slide in the outer tube under the adjustment of the control unit; and the control core wire penetrates through the inner tube, the control core wire has a proximal end connected to the control unit, and the control core wire is configured to slide in the inner tube under the adjustment of the control unit;
a collection net basket having a proximal end fixedly connected to a distal end of the outer tube; and
an adjustable net ball having a proximal end fixedly connected to a distal end of the inner tube and a distal end connected to a proximal end of the guide spring, wherein the control core wire penetrates through the adjustable net ball and has a distal end fixedly connected to the proximal end of the guide spring, so that the adjustable net ball can expand or contract under the action of the control core wire.

2. The combined adjustable thrombectomy device according to claim 1, wherein the control unit comprises an outer shell and a sliding mechanism; and
an adjusting button is arranged on the outer shell.

3. The combined adjustable thrombectomy device according to claim 2, wherein a scale designation is arranged on the outer shell and is configured to display position information of the adjusting button.

4. The combined adjustable thrombectomy device according to claim 2, wherein the sliding mechanism is a direct connecting mechanism, a spiral mechanism, a gear-rack mechanism, a ratchet mechanism or a connecting rod mechanism.

5. The combined adjustable thrombectomy device according to claim 2, wherein an adjusting mode of the adjusting button comprises sliding, rotating or pulling.

6. The combined adjustable thrombectomy device according to claim 2, wherein the adjusting button comprises a first adjusting button and a second adjusting button;
the first adjusting button is connected to the proximal end of the inner tube through the sliding mechanism and is configured to adjust relative positions of the adjustable net ball and the collection net basket; and
the second adjusting button is connected to the proximal end of the control core wire through the sliding mechanism and is configured to adjust expansion or contraction of the adjustable net ball.

7. The combined adjustable thrombectomy device according to claim 1, wherein the collection net basket is a single-layered net ball or a multi-layered net ball; and
a pore diameter of a mesh at the proximal end of the collection net basket is greater than a pore diameter of a mesh at the distal end of the collection net basket.

8. The combined adjustable thrombectomy device according to claim 1, wherein the collection net basket is interwoven by nickel-titanium metal wires or cobalt-chromium alloy wires; and
the collection net basket is cylindrical in a compressed state.

9. The combined adjustable thrombectomy device according to claim 1, wherein the adjustable net ball is interwoven by nickel-titanium metal wires or cobalt-chromium alloy wires;
the adjustable net ball is cylindrical in a compressed state; and
the adjustable net ball is spherical, ellipsoidal, disc-like, cone-shaped, cylindrical or sausage-shaped in an expanded state.

10. The combined adjustable thrombectomy device according to claim 9, wherein the section of the nickel-titanium metal wires is round, elliptical or square.

11. The combined adjustable thrombectomy device according to claim 1, wherein the guide spring is wound by the nickel-titanium alloy wires; and
a ball cap structure is arranged at the distal end of the guide spring.

12. The combined adjustable thrombectomy device according to claim 1, wherein a first developing mark is arranged at the proximal end of the collection net basket;
a second developing mark is arranged at the proximal end of the adjustable net ball; and
a third developing mark is arranged at the distal end of the adjustable net ball.

13. The combined adjustable thrombectomy device according to claim 1, wherein the adjustable net ball is a single net ball or a plurality of net balls connected in series.
